# NEW EUROPEAN PATENT SPECIFICATION

(11) **EP 2 703 035 B2**
(45) Date of publication and mention of the opposition decision: **21.06.2023**
(45) Mention of the grant of the patent: 12.02.2020
(21) Application number: 13181555.7
(22) Date of filing: 23.08.2013
(51) Int. Cl.: A61M 25/00, A61M 25/01

(54) **A packaged catheter assembly**
Verpackte Katheteranordnung
Ensemble de cathéter emballé

(30) Priority: 29.08.2012 AU 2012101310
(43) Date of publication of application: 05.03.2014
(73) Proprietor: Sayco Pty. Ltd., Moorabbin, Victoria 3189 (AU)
(72) Inventor: Young, Tim, Moorabbin, Victoria 3189 (AU)
(74) Representative: Gill Jennings & Every LLP

(56) References cited:
- EP-A1- 2 106 821
- EP-A2- 2 826 514
- WO-A1-98/06642
- WO-A2-2005/014055
- AT-B- 369 994
- DE-A1- 3 223 535
- DE-U1-202011 107 025
- SE-C2- 518 002
- US-A- 4 140 127
- US-A- 5 454 798
- US-A- 5 895 374
- US-A1- 2003 018 322
- US-A1- 2003 168 365
- US-A1- 2005 070 882
- US-B1- 6 217 569
- SE 518002 - Machine translation

## Description

### FIELD OF INVENTION

The present invention relates to a packaged catheter assembly. In particular, the invention relates to a packaged catheter assembly that includes a catheter shaft and catheter sleeve, these components being arranged in the package to facilitate handling of the catheter assembly in use.

### BACKGROUND ART

Urinary catheters are known and generally include a latex, polyurethane, or silicone tube that is inserted into a patient's bladder via the urethra. Catheterisation allows the patient's urine to drain freely from the bladder for collection. Urinary catheters may also be used to inject liquids used for treatment or diagnosis of bladder conditions. A clinician or nurse usually performs the catheterisation procedure, but self-catheterisation is also possible. If long term catheterisation is needed, catheters that facilitate self-catheterisation are most desirable to give patients a greater degree of independence.

Urinary catheters may be permanent (indwelling catheters), or may be intermittent and removed after each catheterisation. A Foley catheter (indwelling urinary catheter) is retained by means of a balloon at the tip of the catheter that is inflated with sterile water. The balloons typically come in two different sizes: 5 cm³ and 30 cm³ and are commonly made in silicone rubber or natural rubber. An intermittent catheter, or Robinson catheter, is a flexible catheter used for short term drainage of urine. Unlike the Foley catheter, it has no balloon on its tip and therefore cannot stay in place unaided. These can be non-coated or coated, for example they may be coated with a hydrophilic coating, and are generally packaged ready for use. Coude catheters are designed with a curved tip that makes it easier to pass the catheter through the curvature of the prostatic urethra. Hematuria (or haematuria) catheters are a type of Foley catheter used for Post-TURP hemostasis. This is useful following endoscopic surgical procedures, or in the case of gross hematuria. There are both two-way and three-way hematuria catheters (i.e. double and triple lumen). External, Texas, urisheath, and condom catheters are generally used for incontinent males and carry a lower risk of infection than an indwelling catheter.

For some patients the insertion and removal of a catheter causes discomfort or pain, so a topical anesthetic is sometimes used. As mentioned above, in many cases catheterisation is performed as a sterile medical procedure by trained, qualified personnel, using equipment designed for this purpose. In the case of intermittent self-catheterisation, patients are trained to perform the procedure themselves. Intermittent self-catheterisation is performed by the patient four to six times a day, using a clean technique in most cases. Incorrect technique may cause trauma to the urethra or prostate (male), urinary tract infection, or a paraphimosis in the uncircumcised male. For patients with spinal cord lesions and neurogenic bladder dysfunction, intermittent catheterisation (IC) is a standard method for bladder emptying. The technique is safe and effective and results in improved kidney and upper urinary tract status, lessening of vesicoureteral reflux and amelioration of continence. In addition to the clinical benefits, patient quality of life is enhanced by the increased independence and security offered by self-catheterisation.

In order to maximise the potential for sterile insertion of catheters and thereby minimise the chances of urinary tract infection during self-catheterisation, a number of designs for catheter packaging have been proposed. The designs generally aim to remove the need for direct physical contact with the catheter during insertion. For example, in some instances the catheter is packaged within a bag from which it is fed by a user via indirect contact through the bag. In this example, the bag may contain a lubricant to assist with feeding of the catheter. In other examples, the catheter is removable from a package and is provided with a sleeve through which the catheter may be fed. In this example, a user pinches the sleeve to indirectly engage the catheter and facilitate insertion.

The options that are currently available are generally relatively easy to use when the user or person inserting the catheter has full function and control of their hands. However, in many instances users of catheters have reduced or limited hand and/or arm function. In such cases, catheters provided with packaging and sleeves as discussed above can be difficult to insert.

International patent application publication number WO 2005/014055 discloses a vapour hydrated hydrophilic catheter assembly and method of manufacturing such assemblies. The catheter assembly includes a gas impermeable package housing a catheter within a cavity. The catheter has a hydrophilic coating applied to at least a portion of its outer surface. The catheter assembly is disposed within the cavity together with a limited amount of a vapour donating liquid provided to activate the hydrophilic coating. The activation of at least part of the hydrophilic coating occurs through exposure of the catheter to a vapour produced by the vapour donating liquid.

Austrian patent application number 717 178 discloses an aseptic urethral catheter unit. The unit includes a receptacle and a catheter. Between opposed side edges of the receptacle, two opposed truncated angles define a lower chamber and an upper chamber. The angles further define a central opening which surrounds a tubular guide. In order to facilitate the passage of the catheter through the guide, a lubricant may be used.

United States patent number 6,217,569 discloses a flexible, tubular envelope, termed a "shroud", for sheathing a catheter. The shroud, passing over the entire catheter, is partially disposable in the distal end of the catheter and is extractable therefrom by manipulation of the user. The shroud terminates in a closed end that includes an integral collector, a pouch/container, which has no specific definition. Devices are integrated into the shroud to aid in its manipulation, as well as in the later separation and sealing of the collector.

The subject matter claimed herein is not limited to embodiments that solve any disadvantages or that operate only in environments such as those described above. Rather, this background is only provided to illustrate one exemplary technology area where some embodiments described herein may be practice.

### SUMMARY OF INVENTION

The present invention relates to a packaged catheter assembly in accordance with the appended claims.

The packaging is openable from one end. The packaging comprises an elongate sleeve within which the catheter assembly is packaged, the elongate sleeve comprising two opposed elongate sheets of packaging material joined along edges thereof. The elongate sleeve comprises a pull tab at the openable end thereof, the pull tab facilitating opening of the elongate sleeve. For example, the pull tab may include an aperture adapted to receive a finger or thumb of a user to facilitate opening of the elongate sleeve. Sides of the opposed sheets are adapted to part from each other on the application of force by the user, thereby facilitating opening of the elongate sleeve.

The form of the catheter shaft is not particularly limited and may include any conventional or future catheter shaft. In any case, the catheter shaft includes at least one inlet at the distal end. Generally, the catheter shaft will include a plurality of inlets at its distal end.

The catheter sleeve may take any suitable form. For example, the catheter sleeve in its simplest form may comprise a tubular body portion and a head portion, the catheter sleeve extending circumferentially around the catheter shaft with the head portion being orientated towards the distal end of the catheter shaft. In addition, the catheter sleeve may comprise gripping surfaces on opposing sides thereof, the gripping surfaces being provided with texture for facilitating gripping of the catheter shaft with the catheter sleeve.

In certain embodiments it may be advantageous for a lubricant to be included. As such, the packaged catheter assembly may further comprise a lubricant within the packaging which lubricates the distal end of the catheter shaft.

The present invention consists of features and a combination of parts hereinafter fully described and illustrated in the accompanying drawings, it being understood that various changes in the details may be made without departing from the scope of the invention or sacrificing any of the advantages of the present invention.

### BRIEF DESCRIPTION OF ACCOMPANYING DRAWINGS

To further clarify various aspects of some embodiments of the present invention, a more particular description of the invention will be rendered by references to specific embodiments thereof, which are illustrated in the appended drawings. It is appreciated that these drawings depict only typical embodiments of the invention and are therefore not to be considered limiting of its scope. The invention will be described and explained with additional specificity and detail through the accompanying drawings in which:

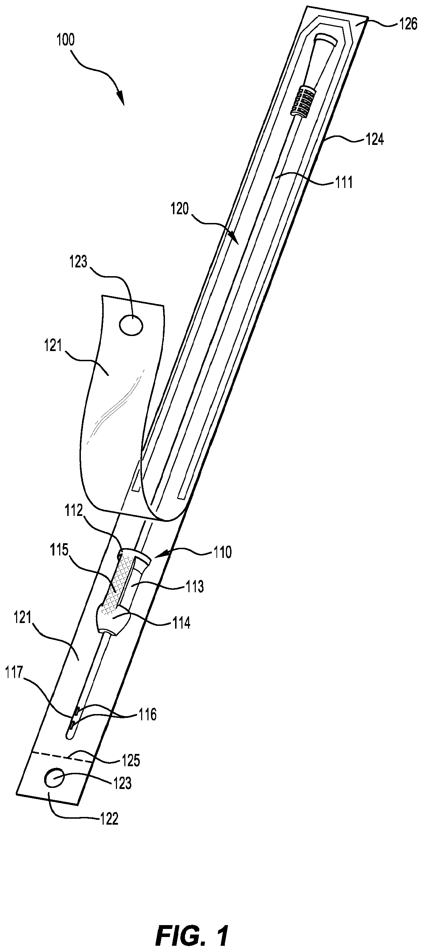

Figure 1 illustrates the packaged catheter assembly of an embodiment of the invention.

### DETAILED DESCRIPTION OF THE PREFERRED EMBODIMENTS

The present invention provides a packaged catheter assembly. In particular, the invention relates to a packaged catheter assembly that includes a catheter shaft and catheter sleeve, these components being arranged in the package to facilitate handling of the catheter assembly in use.

Hereinafter, this specification will describe the present invention according to the preferred embodiments. It is to be understood that limiting the description to the preferred embodiments of the invention is merely to facilitate discussion of the present invention and it is envisioned without departing from the scope of the appended claims.

Referring to Figure 1, a packaged catheter assembly 100 is provided. The packaged catheter assembly 100 includes a catheter assembly 110 and packaging 120. The form of the catheter assembly 110 is not particularly limited. In this embodiment, the catheter assembly includes a catheter shaft 111 and a catheter sleeve 112. The catheter sleeve 112 is tubular and extends circumferentially around the catheter shaft 111. In the illustrated embodiment, the catheter sleeve includes a body portion 113 and a head portion 114, although many other forms may be suitable. The body portion 113 is provided with opposed textured gripping surfaces 115 which facilitate indirect engagement of the catheter shaft 111 by a user of the catheter assembly 110.

The catheter shaft includes a plurality of inlets 116 at its distal end 117 which is to be inserted into the user of the catheter assembly 110. The catheter sleeve 112 is disposed on the distal end 117 of the catheter shaft 111, that is, the end of the catheter shaft 111 that is to be inserted into a user of the catheter assembly 110. Both the distal end 117 and the catheter sleeve 112 are disposed at one end of the packaged catheter assembly 100.

The packaging 120 includes two opposed elongate sheets 121, which are generally rectangular in shape, although other embodiments may be equally applicable. The opposed elongate sheets 121 are separable, and therefore the packaging 120 is openable at one end thereof 122. To assist opening of the packaging 120, the elongate sheets 121 are provided with apertures 123 which may receive a finger or thumb of a user to facilitate opening of the packaging 120. Advantageously, this may assist those users who have limited hand or arm movement.

The sides 124 of the elongate sheets 121 are joined, but can be easily parted by a user on the application of force, thereby enabling access to the catheter assembly 110. The sides 124 of the elongate sheets 121 will not be joined at the openable end 122. Rather, a join will be provided at a location 125 traversing the openable end 122 such that the apertures 123 can be gripped by a user to facilitate opening of the packaging 120. At the other end 126 of the packaging 120, the elongate sheets are permanently joined.

In use, a user of the catheter assembly 110 opens the packaging 120 by gripping the apertures 123 and tearing the elongate sheets 121 from each other along joined sides 124. This facilitates access to the catheter sleeve 112 and insertion of the distal end 117 of the catheter shaft 111 into the user. There is no need for manipulation of the catheter shaft 111 or catheter sleeve 112 as the distal end 117 of the catheter shaft 111 and the catheter sleeve 112 which indirectly engages the catheter shaft 111 are located at the openable end 112 of the packaging 120. This provides substantial advantages to the user of the catheter assembly 110, in particular those have limited hand or arm movement.

Unless the context requires otherwise or specifically stated to the contrary, integers, steps or elements of the invention recited herein as singular integers, steps or elements clearly encompass both singular and plural forms of the recited integers, steps or elements.

Throughout this specification, unless the context requires otherwise, the word "comprise", or variations such as "comprises" or "comprising", will be understood to imply the inclusion of a stated step or element or integer or group of steps or elements or integers, but not the exclusion of any other step or element or integer or group of steps, elements or integers. Thus, in the context of this specification, the term "comprising" is used in an inclusive sense and thus should be understood as meaning "including principally, but not necessarily solely".

It will be appreciated that the foregoing description has been given by way of illustrative example of the invention and that all such modifications and variations thereto as would be apparent to persons of skill in the art are deemed to fall within the scope of the invention as set forth in the appended claims.

## Claims

1. A packaged catheter assembly (100) comprising:
a catheter assembly (110) comprising a catheter shaft (111) and a catheter sleeve (112) mounted on said catheter shaft; and
packaging (120) for said catheter assembly, in which said catheter assembly is packaged,
said packaging being openable from one end thereof (122), said catheter sleeve being mounted towards a distal end (117) of said catheter shaft, said distal end of said catheter shaft and said catheter sleeve being disposed at or towards said openable end of said packaging,
**characterised in that**:
said catheter shaft (111) extends through said catheter sleeve (112); and
said catheter sleeve is formed from a compressible resilient material and can be compressed by a user to indirectly engage said catheter shaft without physical contact between said user and said catheter shaft, thereby facilitating insertion of the distal end (117) of said catheter shaft (111) into said user;
the packaged catheter assembly further **characterised in that** said packaging comprises an elongate sleeve within which said catheter assembly is packaged, said elongate sleeve comprising two opposed elongate sheets (121) of packaging material joined along edges thereof, and **in that** said elongate sleeve comprises a pull tab at said openable end thereof, said pull tab facilitating opening of said elongate sleeve;
wherein said elongate sheets are separable and wherein the sides (124) of the elongate sheets are not joined at the openable end of the packaging, wherein a join is provided at a location (125) traversing the openable end,
and wherein, at the other end (126) of the packaging, the elongate sheets are permanently joined.

2. A packaged catheter assembly according to claim 1, further **characterised in that** said pull tab includes an aperture (123) adapted to receive a finger or thumb of a user to facilitate opening of said elongate sleeve.

3. A packaged catheter assembly according to any one of the preceding claims, further **characterised in that** said catheter shaft includes at least one inlet (116) at said distal end (117).

4. A packaged catheter assembly according to any one of the preceding claims, further **characterised in that** said catheter sleeve comprises a tubular body portion (113) and a head portion (114), said catheter sleeve extending circumferentially around said catheter shaft with said head portion being orientated towards said distal end of said catheter shaft.

5. A packaged catheter assembly according to claim 1, further **characterised in that** said catheter sleeve comprises gripping surfaces (115) on opposing sides thereof, said gripping surfaces being provided with texture for facilitating gripping of said catheter shaft.

6. A packaged catheter assembly according to any one of the preceding claims, further **characterised by** a lubricant within said packaging which lubricates said distal end of said catheter shaft.

## Patentansprüche

1. Verpackte Katheteranordnung (100), umfassend:
eine Katheteranordnung (110), umfassend einen Katheterschaft (111) und eine auf dem Katheterschaft angebrachte Katheterhülse (112); und
eine Verpackung (120) für die Katheteranordnung, in der die Katheteranordnung verpackt ist,
wobei die Verpackung von einem Ende davon (122) geöffnet werden kann, wobei die Katheterhülse zu dem distalen Ende (117) des Katheterschafts hin angebracht ist, wobei das distale Ende des Katheterschafts und die Katheterhülse an oder zu dem Ende, das geöffnet werden kann, der Verpackung hin, eingerichtet ist,
**dadurch gekennzeichnet, dass:**
sich der Katheterschaft (111) durch die Katheterhülse (112) erstreckt; und
die Katheterhülse aus einem komprimierbaren elastischen Material ausgebildet ist und durch einen Benutzer komprimiert werden kann, um den Katheterschaft ohne physischen Kontakt zwischen dem Benutzer und dem Katheterschaft indirekt in Eingriff zu nehmen, wodurch das Einführen des distalen Endes (117) des Katheterschafts (111) in den Benutzer erleichtert wird;
wobei die verpackte Katheteranordnung ferner **dadurch gekennzeichnet ist, dass** die Verpackung eine längliche Hülse umfasst, in der die Katheteranordnung verpackt ist, die längliche Hülse umfassend zwei gegenüberliegende längliche Blätter (121) aus Verpackungsmaterial, die entlang von Rändern davon verbunden sind, und dass die längliche Hülse eine Zuglasche an dem Ende davon, das geöffnet werden kann, umfasst, wobei die Zuglasche das Öffnen der länglichen Hülse erleichtert;
wobei die länglichen Blätter trennbar sind, und wobei die Seiten (124) der länglichen Blätter nicht an dem Ende, das geöffnet werden kann, der Verpackung verbunden sind, wobei eine Verbindung an einer Stelle (125) bereitgestellt ist, die das Ende, das geöffnet werden kann, durchquert,
und wobei an dem anderen Ende (126) der Verpackung die länglichen Blätter dauerhaft verbunden sind.

2. Verpackte Katheteranordnung nach Anspruch 1, ferner
**dadurch gekennzeichnet, dass** die Zuglasche eine Öffnung (123) einschließt, die angepasst ist, um einen Finger oder Daumen eines Benutzers aufzunehmen, um das Öffnen der länglichen Hülse zu erleichtern.

3. Verpackte Katheteranordnung nach einem der vorstehenden Ansprüche, ferner **dadurch gekennzeichnet, dass** der Katheterschaft mindestens einen Einlass (116) an dem distalen Ende (117) einschließt.

4. Verpackte Katheteranordnung nach einem der vorstehenden Ansprüche, ferner **dadurch gekennzeichnet, dass** die Katheterhülse einen rohrförmigen Körperabschnitt (113) und einen Kopfabschnitt (114) umfasst, wobei sich die Katheterhülse in Umfangsrichtung um den Katheterschaft herum erstreckt, wobei der Kopfabschnitt zu dem distalen Ende des Katheterschafts hin ausgerichtet ist.

5. Verpackte Katheteranordnung nach Anspruch 1, ferner
**dadurch gekennzeichnet, dass** die Katheterhülse auf gegenüberliegenden Seiten davon Greifoberflächen (115) aufweist, wobei die Greifoberflächen mit einer Textur zum Erleichtern des Greifens des Katheterschafts versehen sind.

6. Verpackte Katheteranordnung nach einem der vorstehenden Ansprüche, ferner **gekennzeichnet durch** ein Schmiermittel innerhalb der Verpackung, das das distale Ende des Katheterschafts schmiert.

## Revendications

1. Ensemble de cathéter emballé (100) comprenant :
un ensemble de cathéter (110) comprenant une tige de cathéter (111) et un manchon de cathéter (112) monté sur ladite tige de cathéter ; et
l'emballage (120) pour ledit ensemble de cathéter, ledit ensemble cathéter étant emballé,
ledit emballage pouvant être ouvert à partir d'une extrémité de celui-ci (122), ledit manchon de cathéter étant monté vers une extrémité distale (117) de ladite tige de cathéter, ladite extrémité distale de ladite tige de cathéter et dudit manchon de cathéter étant disposés au niveau ou en direction de ladite extrémité ouvrable dudit emballage,
**caractérisé en ce que** :
ladite tige de cathéter (111) s'étend à travers ledit manchon de cathéter (112) ; et
ledit manchon de cathéter est formé à partir d'un matériau élastique compressible et peut être comprimé par un utilisateur pour mettre en prise indirectement ladite tige de cathéter sans contact physique entre ledit utilisateur et ladite tige de cathéter, facilitant ainsi l'insertion de l'extrémité distale (117) de ladite tige de cathéter (111) dans ledit utilisateur ;
l'ensemble de cathéter emballé étant en outre **caractérisé en ce que** ledit emballage comprend un manchon allongé à l'intérieur duquel ledit ensemble de cathéter est emballé, ledit manchon allongé comprenant deux feuilles allongées opposées (121) de matériau d'emballage jointes le long des bords de celles-ci, et **en ce que** ledit manchon allongé comprend une languette de traction au niveau de ladite extrémité ouvrable de celui-ci, ladite languette de traction facilitant l'ouverture dudit manchon allongé ;
lesdites feuilles allongées étant séparables et les côtés (124) des feuilles allongées n'étant pas joints au niveau de l'extrémité ouvrable de l'emballage, un joint étant fourni au niveau d'un emplacement (125) traversant l'extrémité ouvrable,
et, à l'autre extrémité (126) de l'emballage, les feuilles allongées étant jointes de manière permanente.

2. Ensemble de cathéter emballé selon la revendication 1, **caractérisé en outre en ce que** ladite languette de traction comporte une ouverture (123) adaptée pour recevoir un doigt ou un pouce d'un utilisateur afin de faciliter l'ouverture dudit manchon allongé.

3. Ensemble de cathéter emballé selon l'une quelconque des revendications précédentes, **caractérisé en outre en ce que** ladite tige de cathéter comporte au moins une entrée (116) au niveau de ladite extrémité distale (117).

4. Ensemble de cathéter emballé selon l'une quelconque des revendications précédentes, **caractérisé en outre en ce que** ledit manchon de cathéter comprend une partie de corps tubulaire (113) et une partie de tête (114), ledit manchon de cathéter s'étendant circonférentiellement autour de ladite tige de cathéter, ladite partie de tête étant orientée vers ladite extrémité distale de ladite tige de cathéter.

5. Ensemble de cathéter emballé selon la revendication 1, **caractérisé en outre en ce que** ledit manchon de cathéter comprend des surfaces de préhension (115) sur les côtés opposés de celui-ci, lesdites surfaces de préhension étant dotées d'une texture pour faciliter la préhension de ladite tige de cathéter.

6. Ensemble de cathéter emballé selon l'une quelconque des revendications précédentes, **caractérisé en outre par** un lubrifiant à l'intérieur dudit emballage qui lubrifie ladite extrémité distale de ladite tige de cathéter.
